# EUROPEAN PATENT SPECIFICATION

(11) **EP 4 497 467 B1**
(45) Date of publication and mention of the grant of the patent: **16.09.2026**
(21) Application number: 23854108.0
(22) Date of filing: 28.06.2023
(51) Int. Cl.: A61M 60/13, A61M 60/414, A61M 60/416, A61M 60/81, A61M 60/857, A61M 60/237

(54) **INTERVENTIONAL BLOOD PUMP**
INTERVENTIONELLE BLUTPUMPE
POMPE D'ASSISTANCE CIRCULATOIRE INTERVENTIONNELLE

(30) Priority: 18.08.2022 CN 202210991439; 18.08.2022 CN 202222177977 U
(43) Date of publication of application: 29.01.2025
(73) Proprietor: RocketHeart Technology Co. LTD, Tianjin 300453 (CN)
(72) Inventor: HAN, Zhifu, Tianjin 300453 (CN); ZHANG, Xuman, Tianjin 300453 (CN); FAN, Qinglin, Tianjin 300453 (CN); WANG, Xian, Tianjin 300453 (CN); WANG, Chao, Tianjin 300453 (CN); ZHANG, Hongqing, Tianjin 300453 (CN); MA, Hongbin, Tianjin 300453 (CN); DING, Mingqian, Tianjin 300453 (CN); JING, Pengfei, Tianjin 300453 (CN)
(74) Representative: Zacco GmbH
(86) International application number: PCT/CN2023/103393
(87) International publication number: WO 2024/037203

(56) References cited:
- WO-A1-00/43053
- CN-A- 113 599 692
- CN-A- 114 082 098
- CN-A- 114 129 890
- CN-A- 114 259 645
- CN-A- 115 151 299
- CN-U- 219 050 101
- US-A1- 2019 046 703
- US-A1- 2021 213 273

## Description

### Technical Field

The present invention relates to a field of medical devices, and in particular, to an interventional blood pump that is percutaneously inserted into a blood vessel of a patient.

### Background

An interventional catheter pump, also known as an interventional blood pump, is mostly used for high-risk Percutaneous Coronary Intervention (PCI) to reduce ventricular work and provides necessary circulatory support for cardiac recovery and early assessment of a residual myocardial function. The most mature and advanced interventional catheter pump currently available in the world is the Impella series developed by AbioMed. This type of blood pumping assist device is introduced into the heart of a patient through blood vessels. During operation, an inlet of the catheter pump is placed in the ventricle and an outlet is placed in the artery. Blood is pumped from the ventricle into the artery to ensure blood perfusion of the coronary artery and systemic organs of the patient during PCI surgery, thereby reducing the heart load. This catheter pump is generally composed of a catheter, an impeller, a motor, and other components. When the motor drives the impeller to rotate, the blood is delivered from the inlet of the blood pumping catheter to the outlet. At this time, the blood pumping efficiency of the blood pump is a decisive indicator of the performance of the blood pump. However, the catheter of the existing blood pump is too slender, resulting in significant pressure loss and difficulty in improving pumping efficiency of the blood pump, which still needs optimization and improvement.

A known blood pump includes a rotatable impeller for blood delivery, and a mesh housing surrounding the impeller. The impeller and the housing are foldable and can be automatically unfolded after being forcibly compressed, the impeller is connected to the motor placed outside a body of the patient through a flexible driving shaft, a flexible outflow tube is arranged around the flexible driving shaft, and the outflow tube includes an outlet configured to discharge the blood into the artery at a proximal end of the outflow tube. For example, Chinese patent application CN108136089A discloses such a blood pump. In the above solution, the foldable impeller and the impeller housing can be forcibly compressed to a relatively small diameter during intervention, so that a pump head can be guided to a position that is difficult to access, which facilitates the intervention operation and reduces the pain of the patient. However, such a blood pump also has some problems, especially: when a soft shaft drives the impeller to rotate at a high speed, the stability is not good, and after the impeller and an impeller outer cylinder are delivered to an operating position in the blood vessel in a folded state, there is a certain uncertainty as to whether the impeller and the impeller outer cylinder can be fully unfolded according to a predetermined design, which may affect the normal operation of the blood pump.
A blood pump using cross-flow principles is disclosed in Document D1(WO00/43053A1);
An intravascular blood pump with outflow hose is disclosed in Document D2 (US 2021/213273 A1);
A pump to motor connection system is disclosed in Document D3 (US 2019/046703 A1).

### Summary

An embodiment of the present invention is to solve the above technical problems.

An interventional blood pump according to the present invention comprises the technical features as defined in independent claim 1.

The interventional blood pump includes a pump body and a driving unit. The pump body includes a blood inlet and at least one blood outlet. The pump body includes a rotatable rigid impeller configured to provide power for the flow of blood and accommodated in a rigid impeller outer cylinder. A proximal end of the impeller is connected to a distal end of the driving unit through a flexible shaft, a distal end of the impeller outer cylinder communicates with the blood inlet, and a proximal end of the impeller outer cylinder includes an impeller outlet. The pump body further includes a radially expandable catheter arranged outside the flexible shaft and extending in at least one part of the length of the flexible shaft. A distal end of the radially expandable catheter is hermetically connected to the impeller outer cylinder and covers the impeller outlet, and a proximal end of the radially expandable catheter is provided with the blood outlet. When the radially expandable catheter is in a radial expansion state, a gap between the radially expandable catheter and the flexible shaft forms a blood flow channel communicating with the blood inlet and the blood outlet. When the pump body is at an operating position in the heart, the blood inlet is located in the left ventricle or right ventricle, the blood outlet is located in the aorta or pulmonary artery, and the radially expandable catheter spans the corresponding artery flap. First, the inventor of the present invention has found through research that the mesh outer cylinder and the foldable impeller in the related art need to be fixed to a transmission soft shaft passing through them, so that the stability is not good when the soft shaft drives the impeller to rotate at a high speed. In the above solution of the embodiment of the present invention, since the rigid impeller is rotatably fixed in the rigid impeller outer cylinder, the radial position relationship between the impeller and the impeller outer cylinder is fixed, which can ensure that the impeller rotates stably at a high speed in the impeller outer cylinder. Secondly, since the impeller is connected to the driving unit through the flexible shaft instead of being directly connected thereto, the length of a rigid section in a blood vessel is reduced, the operability is improved, and the position of the driving unit may be set more flexibly. Thirdly, since the radially expandable catheter is arranged around the flexible shaft, and the distal end of the radially expandable catheter is hermetically connected to the impeller outer cylinder and covers the impeller outlet, and the proximal end of the radially expandable catheter is provided with the blood outlet, the radially expandable catheter may be contracted to a very small outer diameter during intervention and be tightly attached to the flexible shaft, thereby reducing the damage to the blood vessel and the incidence of vascular complications during intervention of the pump body, and facilitating postoperative recovery of a patient. However, when the blood pump operates, the inner diameter of the expandable catheter is expanded, and the blood flow channel with a relatively large cross-section is formed between the expandable catheter and the flexible shaft, thereby ensuring the flow area of the blood. In addition, since the radially expandable catheter is clamped in a middle of the artery flap in an operating state, as the heart beats, the flexible catheter is squeezed through the opening and closing of the valve, so that the flow in the catheter forms a certain pulsating flow. This blood pumping manner is more in line with the physiological structure of a human body. Moreover, when the impeller operates, the blood flowing out from the impeller outlet does not flow directly into the artery, but flows into the blood flow channel between the radially expandable catheter and the flexible shaft after expansion, and then enters the artery from the blood outlets at the proximal end of the radially expandable catheter. Research has found that such an arrangement prevents the impeller outlet from dissipating outlet energy due to blood flow chaos, thereby improving the operating efficiency of the entire pump.

According to some embodiments of the present invention, in the above interventional blood pump, the length of the flexible shaft is set, so that when the pump body is at the operating position in the heart, the driving unit is located in the aorta or pulmonary artery. In the related art, there is a technical solution that places the driving unit outside the body. In this solution, the problems of size and heat dissipation of the driving unit are ignored, but a very long flexible shaft (up to 1500 mm in length) is needed to connect the impeller and the driving unit. However, the manufacturing and connection process of the too long flexible shaft is complicated, and the mechanical properties need to meet very high requirements, which reduce the transmission efficiency of the driving unit. In addition, after the pump body is intervened, the long flexible shaft may be in a 180-degree U-shaped bent state in the blood vessel. In the bent state, the high-speed rotation of the soft shaft has the risk of wear and fracture of the flexible shaft, and even damage to a blood vessel wall. There are precedents for this internationally. According to the solution of the embodiment of the present invention, the length of the flexible shaft connecting the impeller and the driving unit is greatly shortened by building the driving unit into the body of the patient, thereby avoiding the above various problems.

In some embodiments, the driving unit is arranged outside the blood outlets at the proximal end of the radially expandable catheter and adjacent to the blood outlets, which means that the length of the flexible shaft is substantially equivalent to the length of the radially expandable catheter, the radially expandable catheter needs to be transvalvular (together with the flexible shaft arranged therein), and a distance between the blood inlet adjacent to the distal end of the radially expandable catheter and each of the blood outlets arranged on the proximal end of the radially expandable catheter may not be too long, so that the length of the flexible shaft is further shortened accordingly. When the blood pump operates, the flexible shaft only exists in a transvalvular section, and a bending radius of the transvalvular section is relatively large, which greatly reduces the risk of wear and fracture of the flexible shaft, and even damage to the blood vessel wall. In addition, since the driving unit is arranged in the blood flow direction outside the blood outlets, when the blood flows along an outer surface of the driving unit, heat generated by the operation of the driving unit is taken away and effectively dissipated, thereby reducing the damage of a heating component to the blood.

According to some embodiments of the present invention, in the above interventional blood pump, the proximal end of the radially expandable catheter is hermetically connected to a housing of the driving unit. The radially expandable catheter is of a flexible structure, and has a relatively large instability. By connecting the proximal end of the radially expandable catheter to the rigid housing of the driving unit and the distal end of the radially expandable catheter to the rigid impeller outer cylinder, both ends are supported by the rigid structures, thereby improving the stability.

According to the present invention, in the above interventional blood pump, the pump body further includes a plurality of rigid outlet brackets connected to the proximal end of the impeller outer cylinder. The outlet brackets define the impeller outlet. When the impeller rotates, the blood entering from the blood inlet flows through the gap between the impeller and the impeller outer cylinder, flows into the blood flow channel through the impeller outlet, and flows out from the blood outlets. The rigid outlet brackets can provide good support for the impeller outer cylinder and define the impeller outlet with a stable shape and a relatively large size, so that the blood can flow smoothly into the blood flow channel. In some embodiments, the outlet brackets are 3 to 5 arc-shaped strips or blades, which are uniformly distributed in a circumferential direction of the impeller outer cylinder.

According to some embodiments of the present invention, in the above interventional blood pump, the pump body further includes a pigtail catheter. A mesh inlet bracket made of a shape memory alloy is arranged between a proximal end of the pigtail catheter and the distal end of the impeller outer cylinder. It is well known that wall suction easily occurs at the blood inlet due to the suction effect of the rotation of the impeller. By adopting the mesh inlet bracket, this problem can be solved to a certain extent. In fact, due to the small gap of the mesh structure and the large elasticity of the material, when the mesh structure is accidentally attached or even sucked to the wall at the pump inlet, the mesh structure will not cause the ventricular wall to fall off into the blood pump, thereby protecting the ventricular wall. In addition, since the inlet bracket is made of the shape memory alloy, it may be forcibly compressed with the help of a sheath tube during intervention to reduce the outer diameter to facilitate the intervention operation. When the blood pump is delivered to the desired operating position, the sheath tube is removed, and the inlet bracket automatically restores the pre-set shape and expands meshes to a normal operating state, thereby allowing the blood to be pumped into the gap between the impeller and the impeller outer cylinder through the meshes.

According to some embodiments of the present invention, in the above interventional blood pump, an outer diameter of the mesh inlet bracket gradually decreases from the distal end to the proximal end and is slightly greater than outer diameters of the impeller outer cylinder and the outlet brackets. In this way, even if a pump head accidentally approaches the ventricular wall, the first contact with the ventricular wall is the distal end part with a relatively large outer diameter of the inlet bracket, rather than the proximal end part with a relatively small diameter close to the impeller, thereby effectively preventing the damage to the ventricular wall caused by impeller suction and the risk that the ventricular wall tissue falls into the blood pump to cause the impeller to stop rotating.

According to some embodiments of the present invention, in the above interventional blood pump, at the proximal end of the mesh inlet bracket, the mesh inlet bracket further includes a rigid ring integrally formed therewith and fixed to the impeller outer cylinder. With the help of the rigid ring, the mesh structure composed of a plurality of metal wires can be more easily and firmly fixed to the rigid impeller outer cylinder. Specifically, the rigid ring may be fixed to an outer surface of the impeller outer cylinder by, for example, welding, bonding, etc.

According to some embodiments of the present invention, in the above interventional blood pump, the pump body further includes a bearing seat fixed to a proximal end of the outlet bracket, a bearing is arranged in the bearing seat. The impeller includes an impeller body, rigid blades arranged on an outer surface of the impeller body, and a rigid impeller shaft extending from the impeller body to the proximal end, the impeller shaft is rotatably arranged in an inner ring of the bearing in a penetrating manner. Since the bearing seat is fixed to the rigid impeller outer cylinder through the rigid outlet brackets, and forms a rigid whole with the impeller outer cylinder, and the impeller shaft of the impeller is rotatably supported in the bearing in the bearing seat, a relative radial position of the rigid impeller and the impeller outer cylinder is fixed, and radial vibration is less likely to occur, thereby improving the rotational stability of the impeller.

According to some embodiments of the present invention, in the above interventional blood pump, the impeller body, the blades, and the impeller shaft are integrally formed. Such an impeller has no joint between various parts, and is relatively high in mechanical strength, more durable, and easier to manufacture. For example, such an integrated impeller is machined by an injection molding or 3D printing process.

According to some embodiments of the present invention, in the above interventional blood pump, the blades are made of implant grade metal materials or implant grade plastics. The implant grade metal materials include, but are not limited to, pure titanium, a titanium alloy, and stainless steel. The implant grade plastics include polyetheretherketone, polycarbonate or polyethylene, etc. "Implantable grade" means that the material is biocompatible, meets relevant national standards, and can be implanted in the body.

According to some embodiments of the present invention, in the above interventional blood pump, the length of the flexible shaft is 50 mm to 80 mm. Those skilled in the art easily understand that the length of the flexible shaft only needs to meet the requirements for transvalvular blood pumping. Under this premise, the shorter the better, so as to avoid various potential risks caused by the too long flexible shaft. According to the observation of the inventor of the present invention, when the length of the flexible shaft is within the above range, the transmission efficiency is relatively high, the risk of wear and fracture caused by the high-speed rotation of the flexible shaft is relatively low, and the requirements for transvalvular blood pumping can be well met.

According to the present invention, in the above interventional blood pump, the flexible shaft includes a soft shaft, a flat wire spring tube sleeved outside the soft shaft, and a sealing hose sleeved outside the flat wire spring tube. An inner diameter of the flat wire spring tube is greater than an outer diameter of the soft shaft, and an outer diameter of the flat wire spring tube is smaller than an inner diameter of the sealing hose. During operation of the blood pump, the soft shaft needs to rotate at a high speed, with a maximum speed of nearly 50,000 rpm. When the flexible shaft is bent, the soft shaft may wear the sealing hose and even damage human tissue. By arranging the flat wire spring tube outside the soft shaft, a good protection effect can be achieved to prevent such very dangerous consequences. At the same time, the flat wire spring tube achieves a certain rigid support effect outside the soft shaft, thereby reducing the vibration and swing of the soft shaft during operation. The sealing hose in the outermost layer can seal lubricating fluid for lubricating and cooling the high-speed rotating soft shaft in the hose to ensure the normal operation of the soft shaft.

According to some embodiments of the present invention, in the above interventional blood pump, a distal end of the soft shaft is fixed to the impeller shaft, and a proximal end of the soft shaft is fixed to an output shaft of the driving unit. The soft shaft of a flexible structure has relatively large instability, and is more likely to vibrate when rotating at a high speed. After the distal end of the soft shaft is connected to the rigid impeller through the impeller shaft, the stability of a shaft system is increased by the bearing in the bearing seat. At the same time, since the proximal end of the soft shaft is also fixed to the rigid body, that is, the output shaft of the driving unit, both ends of the soft shaft are supported by the rigid structures, which provides better stability during high-speed rotation.

According to **the** present invention, in the above interventional blood pump, the pump body further includes a tubular connector fixed to the proximal ends of the outlet brackets. The tubular connector has a distal end region and a proximal end region, an outer diameter of the distal end region is greater than an outer diameter of the proximal end region, the distal end region is connected to a distal end of the sealing hose, and the proximal end region is connected to a distal end of the flat wire spring tube. In this way, the distal ends of the sealing hose and the flat wire spring tube of the flexible shaft are connected to the rigid impeller outer cylinder through the tubular connector, and the distal end of the soft shaft in the flexible shaft is connected to the rigid impeller, so that the entire distal end of the flexible shaft is connected to the rigid body, which can be well supported and improve the stability.

According to **the** present invention, in the above interventional blood pump, the driving unit is a motor, and the motor includes a distal end cover extension section and a motor connector connecting the distal end cover extension section and a proximal end of the flat wire spring tube. The length of the motor connector extending from the distal end cover extension section to the distal end can be adjusted, and a proximal end of the sealing hose is connected to the distal end cover extension section of the motor. In this way, the proximal ends of the flat wire spring tube and the sealing hose are both connected to the rigid body, which further improves the stability of the flexible shaft. In addition, since the length of the motor connector extending from the distal end cover extension section to the distal end can be adjusted, the length relationship between the soft shaft, the flat wire spring tube, and the sealing hose in the flexible shaft may be adjusted according to the assembly length, thereby achieving a firmer and more reliable connection between them and the motor.

According to some embodiments of the present invention, the interventional blood pump further includes a hollow interventional catheter. A distal end of the interventional catheter is connected to a proximal end of the driving unit, and the interventional catheter contains at least a cable for supplying power to the driving unit.

It is to be understood that the above general description and the following detailed description are only exemplary and explanatory and not intended to limit the present invention, which is defined by the appended claims. Other features, purposes, and advantages of the present invention become apparent from the description, the drawings and the claims.

### Brief Description of the Drawings

In order to more clearly illustrate the technical solutions in the embodiments of the present invention, the drawings used in the description of the embodiments will be briefly described below. Those skilled in the art easily understand that these drawings are only for illustrative purposes and are not intended to limit the scope of protection of the present invention, which is defined by the appended claims. For illustrative purposes, these drawings may not be drawn completely to scale.
Fig. 1 is a schematic overall structural diagram of an interventional blood pump according to an embodiment of the present invention.
Fig. 2 is a schematic structural diagram of a pump head of a blood pump shown Fig. 1.
Fig. 3 is a schematic axial cross-sectional view of a flexible shaft of a blood pump shown Fig. 1.
Fig. 4 is an enlarged schematic perspective view of a distal end part of a pump head of a blood pump shown in Fig. 1.
Fig. 5 is a schematic enlarged axial cross-sectional view of a distal end part of a pump head of a blood pump shown in Fig. 1.
Fig. 6 is a schematic enlarged axial cross-sectional view of a proximal end part of a pump head of a blood pump shown Fig. 1.

A list of reference signs is as follows.
100, Blood pump; 101, Pump body; 1, Pigtail catheter; 2, Inlet bracket; 21, Mesh part; 22, Rigid ring; 23, Blood inlet; 3, Impeller; 31, Impeller body; 32, Blade; 33, Impeller shaft; 3,4 Impeller outer cylinder; 35, Outlet bracket; 36, Impeller outlet; 37, Bearing seat; 4, Flexible shaft; 41, Soft shaft; 42, Flat wire spring tube; 43, Sealing hose; 5, Radially expandable catheter; 51, Blood outlet; 52, Gap; 6, Motor; 61, Distal end cover extension section; 62, Output shaft; 63, Motor connector; 7, Interventional catheter; 71, Distal end of interventional catheter; 72, Proximal end of interventional catheter; 8, Handle; 9, Bearing; 10, Tubular connector

### Detailed Description of the Embodiments

Exemplary implementations will be described in detail herein, examples of which are shown in the drawings. When the following description refers to the drawings, the same numbers in different drawings represent the same or similar elements unless otherwise indicated. The implementations described in the following exemplary implementations do not represent all implementations consistent with the present invention. Instead, they are merely examples of apparatuses consistent with some aspects of the present invention as detailed in the appended claims.

Unless otherwise defined, technical and scientific terms used herein have the same meaning as commonly understood by those skilled in the art of the present invention. Terms used in the present invention are only for describing the specific embodiments and are not intended to limit the present invention. Singular forms "a/an", "said" and "the" used in the present invention and appended claims are also intended to include plural forms unless other meanings are clearly expressed in the context. It is also to be understood that the term "and/or" as used herein means and includes any or all possible combinations of one or more associated listed items. "Including" or "including" and similar words mean that the elements or objects appearing in front of "including" or "including" cover the elements or objects listed after "including" or "including" and their equivalents, and do not exclude other elements or objects. "Link" or "connect" and similar words are not limited to a physical or mechanical connection, and may include an electrical connection, whether direct or indirect. "Multiple" includes two, equivalent to at least two. It is to be understood that although the terms first, second, third, etc. may be used in the present invention to describe various information, these information should not be limited to these terms. These terms are only used to distinguish information of the same type from each other. For example, without departing from the scope of the present invention, first information may be referred to as second information, and similarly, the second information may also be referred to as the first information.

In some embodiments of the present invention, unless otherwise specified, the terms "proximal end" and "distal end" are relative to an operator of an interventional blood pump. The part of a component close to the operator is a proximal end, and the part far from the operator is a distal end.

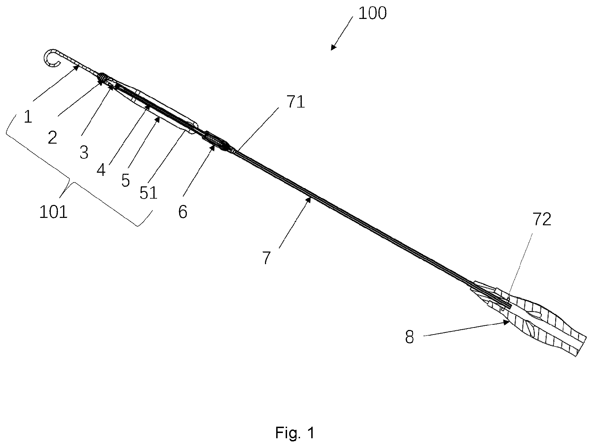

Fig. 1 schematically shows an interventional blood pump 100 according to an embodiment of the present invention. The blood pump may be used as a Ventricular Assist Device (VAD) to assist the ventricle in performing a blood pumping function in high-risk PCI. The blood pump includes a pump body 101 located at a distal end and a driving unit 6 located at a proximal end during operation. In this embodiment, the driving unit 6 is a motor, such as a hollow cup motor. However, it should be understood by those skilled in the art that any driving unit (such as a hydraulic motor) that can output power and is suitable for use in the field of interventional hospitals can be used. In this embodiment, the blood pump 100 further includes a hollow interventional catheter 7, which usually contains a cable for supplying power to the driving unit 6, a lubricating fluid catheter, a sensor optical fiber, etc., and the length thereof may be adjusted according to actual needs, usually up to 1500 mm. A distal end 71 of the interventional catheter 7 is connected to a proximal end of the driving unit 6, and a proximal end 72 of the interventional catheter 7 is connected to a handle 8. During surgical intervention, the interventional catheter 7 achieves a pushing effect. When the blood pump 100 is delivered to an operating position, one part of the interventional catheter 7 is located inside a body and one part is located outside the body, while the handle 8 is located outside the body as a whole, so as to facilitate the operation of a doctor. In some embodiments, like a flexible shaft 4, the interventional catheter 7 is also made of a flexible material and has a bending radius of 25 to 35 mm, and can withstand a U-shaped bend of approximately 180°, so as to better conform to a curved blood vessel during intervention.

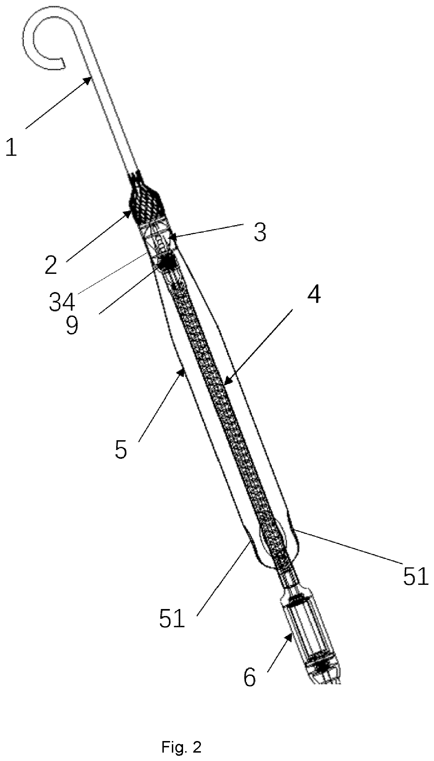

As shown more clearly in Fig. 2, in this embodiment, from the distal end to the proximal end, the pump body 101 sequentially includes a pigtail catheter 1, an inlet bracket 2, a rotatable impeller 3, an impeller outer cylinder 34 for accommodating the impeller 3, the flexible shaft 4, and a radially expandable catheter 5 arranged around the flexible shaft 4. The inlet bracket 2 is provided with a blood inlet 23, and the radially expandable catheter 5 is provided with a blood outlet 51. In some embodiments, a distal end of the pigtail catheter 1 is curved, which can prevent unnecessary damage to the ventricular wall caused by the pump body touching it.

As shown in Fig. 1 and Fig. 2, in this embodiment, the inlet bracket 2 is meshed, and in some embodiments, the inlet bracket 2 is made of a shape memory alloy (such as a nickel-titanium alloy), and is arranged between a proximal end of the pigtail catheter 1 and a distal end of the impeller outer cylinder 34. Since the inlet bracket 2 uses a mesh design, when the pump inlet is close to the ventricular wall or even when the wall is sucked, the mesh structure has small gaps and the material has high elasticity, which can protect the ventricular wall and prevent the ventricular wall from falling off into the blood pump. Specifically, in this embodiment, the inlet bracket 2 includes a mesh part 21 and a rigid ring 22 integrally formed with the mesh part 21. The rigid ring 22 is fixed to an outer surface of the impeller outer cylinder 34 in any suitable manner (e.g., welding, bonding, clamping, etc.). Similarly, a distal end of the mesh part 21 is fixed to the proximal end of the pigtail catheter 1 in any suitable manner, such as being bonded to the outer surface thereof by using a heat shrink tube. The mesh part 21 includes an approximately cylindrical distal end part with a relatively small diameter (i.e., the part sleeved on the pigtail catheter 1, whose diameter is slightly greater than the outer diameter of the proximal end of the pigtail catheter), an approximately cylindrical proximal end part with a relatively large diameter (whose diameter is slightly greater than the outer diameter of the impeller outer cylinder 34, and the rigid ring 22 is arranged at a proximal end of this part), and a truncated conical transition part between the two parts. It should be understood that the inlet bracket 2 may also have any other suitable shape. In some embodiments, the mesh part 21 includes a plurality of meshes (e.g., in an approximately diamond shape) with a width of 0.1 mm to 0.3 mm. These meshes constitute the blood inlet 23. The inlet bracket 2 may be manufactured by braiding metal wires or cutting metal tubes. In some embodiments, the outer diameter of the proximal end part of the inlet bracket 2 gradually decreases from the distal end to the proximal end and is slightly greater than the outer diameters of the impeller outer cylinder 34 and the outlet brackets 35, which can better protect the ventricular wall.

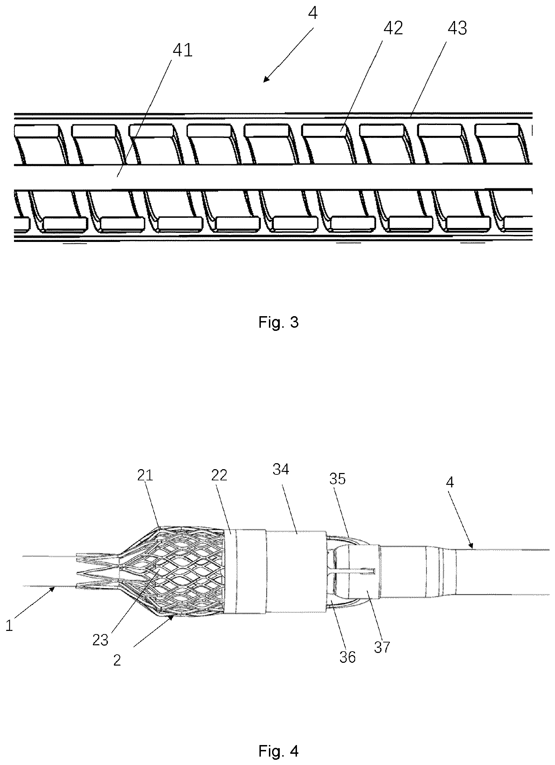

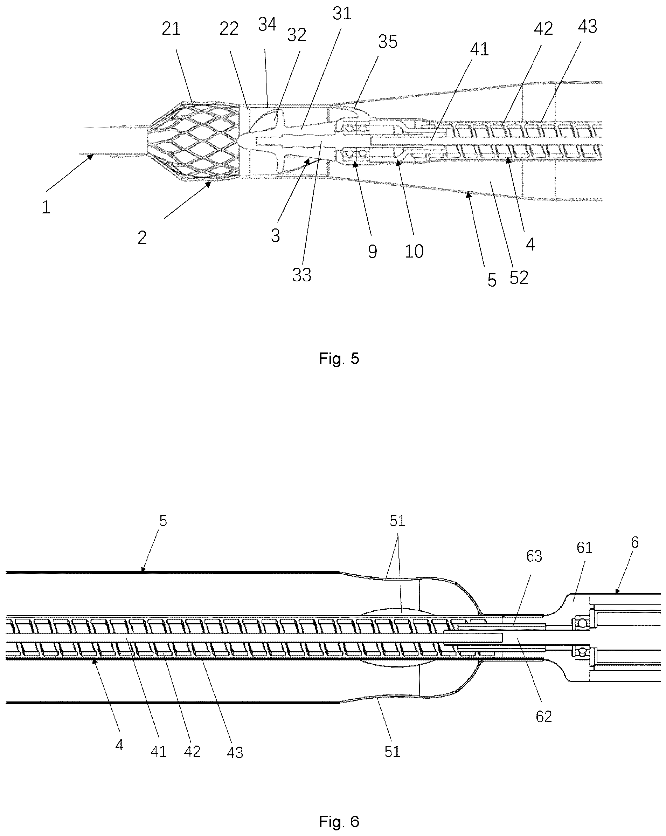

In some embodiments, the impeller 3 is rigid and is accommodated in the rigid impeller outer cylinder 34, and can increase power for the flow of the blood during rotation and assist the ventricle in achieving the blood pumping function. The distal end of the impeller outer cylinder 34 communicates with the blood inlet 23, and the proximal end of the impeller outer cylinder includes an impeller outlet 36 (see Fig. 4). Herein, "rigid" means that no obvious deformation may occur under the action of an external force. In other words, the impeller 3 and the impeller outer cylinder 34 are not foldable, and have basically the same shape in a non-operating state before entering the blood vessel and in a normal operating state thereafter. Since the rigid impeller is rotatably fixed in the rigid impeller outer cylinder, a radial position relationship between the impeller and the impeller outer cylinder is fixed, which can ensure that the impeller rotates stably at a high speed in the impeller outer cylinder. As shown in Fig. 5, in some embodiments, the impeller 3 includes an impeller body 31, rigid blades 32 arranged on an outer surface of the impeller body 31, and a rigid impeller shaft 33 extending from the impeller body 31 to the proximal end, wherein the impeller body 31 is rigid. As described below, the impeller 3 is fixed in the impeller outer cylinder 34 at the proximal end of the impeller by the impeller shaft 33 rotatably arranged in a bearing 9, and the distal end of the impeller is a free end. The impeller body 31, the blades 32, and the impeller shaft 33 may be made of the same or different materials separately, and then assembled together by bonding, welding, etc., or may also be integrally formed. For example, an integrally formed impeller may be manufactured by an injection molding or 3D printing process. In some embodiments, the blades 32 are made of implant grade metal materials or plastics that are biocompatible and can be implanted in the body, such as pure titanium, a titanium alloy, stainless steel, polyetheretherketone, polycarbonate, polyethylene, etc. The impeller 3 should have a suitable diameter. Although the larger the diameter, the more blood may be sucked per unit time, the impeller cannot be too large due to the limitation of the diameter of the blood vessel, otherwise the blood pump cannot be delivered to a target position through the blood vessel. At the same time, the diameter of the impeller 3 cannot be too small, otherwise the blood pumping capacity is too weak, the flow rate is too low, and the machining difficulty is also increased. The impeller outer cylinder 34 is also made of implant grade metal or plastic, usually cylindrical. A gap between a wall of the impeller outer cylinder 34 and the impeller 3 forms a suction flow channel, through which the blood pumped in from the blood inlet 23 first passes. The inner diameter of the impeller outer cylinder 34 needs to be designed in conjunction with the impeller 3, so that the suction flow channel of an appropriate size is formed between the two. When the impeller 3 has a given diameter, the inner diameter of the impeller outer cylinder 4 is too large to reduce the efficiency of the impeller, but the damage effect of the impeller on the blood may also be reduced, so that a balance needs to be found between the two.

As best shown in Fig. 4, in this embodiment, the pump body 101 further includes a plurality of rigid outlet brackets 35 connected to the proximal end of the impeller outer cylinder 34, and the outlet brackets 35 define the impeller outlet 36. The rigid outlet brackets 35 can provide good support for the impeller outer cylinder 34 and define the impeller outlet with a stable shape and a relatively large size, so that the blood can flow smoothly into a blood flow channel (see Fig. 5) formed by the gap 52. The number of outlet brackets 35 should not be too small, otherwise the strength is not large enough and breaks easily, but it should not be too large, otherwise the blood at the impeller outlet 36 may be throttled to affect the flow rate of the blood pump, and the larger the number of outlet brackets, the larger a contact surface between the blood pump and the blood, and the more susceptible to hemolysis. In some embodiments, the outlet brackets 35 are 3 to 5 arc-shaped strips or blades, which are uniformly distributed in a circumferential direction of the impeller outer cylinder 34. In order to facilitate machining and not affect the blood flow field of the impeller outlet, in some embodiments, the width of the outlet brackets 35 is 0.2 to 0.5 mm. As shown in Fig. 4 and Fig. 5, the pump body 101 further includes a bearing seat 37 fixed to proximal ends of the outlet brackets 35, and a bearing 9 is arranged in the bearing seat 37. In some embodiments, the impeller outer cylinder 34, the outlet brackets 35, and the bearing seat 37 are fixed together and form a whole structure, of course, they may also be manufactured separately and then assembled together in any suitable manner.

In the above embodiment, the proximal end of the impeller 3 is connected to a distal end of the driving unit 6 through a relatively short flexible shaft 4, and a length of the flexible shaft 4 is set, so that when the pump body 101 is at the operating position in the heart, the driving unit 6 is located in the aorta or pulmonary artery. It is easy to understand that in such an embodiment, the driving unit 6 should be made of a material suitable for use in the human body, and the size is designed to be small enough, so that the driving unit 6 can pass through the blood vessel smoothly. Since the flexible shaft needs to be transvalvular when the blood pump operates normally, the length cannot be too short, otherwise it cannot meet the transvalvular requirement, nor too long, otherwise it may reduce the transmission efficiency and cause the flexible shaft to be in a bent state in the blood vessel, thereby increasing the risk of wear and fracture of the flexible shaft and even damage to the blood vessel wall that may be caused by the high-speed rotation of the transmission soft shaft. In some embodiments, the length of the flexible shaft is 50 mm to 80 mm. For example, the length of the flexible shaft may be 50 mm, 60 mm, 70 mm, or 80 mm.

Since the blood pump needs to be placed in the body of the patient through the curved blood vessel, the flexible shaft 4 should have a certain elasticity and flexibility, and in some embodiments, the flexible shaft 4 is able to withstand a U-shaped bend of approximately 180 degrees with a bending radius of approximately 30 mm. As shown in Fig. 3, Fig. 5, and Fig. 6, in this embodiment, the flexible shaft 4 includes a flexible shaft 41 for transmitting the torque of the driving unit 6 to the impeller 3 and driving same to rotate at a high speed, a flat wire spring tube 42 sleeved outside the soft shaft 41, and a sealing hose 43 sleeved outside the flat wire spring tube 42. An inner diameter of the flat wire spring tube 42 is greater than an outer diameter of the flexible shaft 41, and an outer diameter of the flat wire spring tube 42 is smaller than an inner diameter of the sealing hose 43. The flexible shaft 41 is usually made by weaving a plurality of (for example, 2 to 6) metal ropes, which may be solid or hollow, and in some embodiments, the diameter of the soft shaft is 0.5 mm to 1 mm. The flat wire spring tube 42 is usually formed by spirally winding a flat wire with a thickness of 0.25 to 0.55 mm in a certain rotation direction. In some embodiments, the winding rotation direction of the flat wire is opposite to the rotation direction of the soft shaft 41, and after winding, a certain axial gap is ensured between adjacent flat wires, which may make the spring have better elasticity and bending flexibility. Since the soft shaft 41 needs to rotate at a high speed during operation of the blood pump, with a maximum speed of nearly 50,000 rpm, the soft shaft 41 may rub against the outer skin when the flexible shaft 4 is bent, so that the flat wire spring tube 42 achieves a protection effect outside the soft shaft 41, thereby preventing the soft shaft 41 from wearing the sealing hose 43 during high-speed rotation and even damaging human tissue. At the same time, the flat wire spring tube 42 achieves a certain rigid support effect outside the soft shaft 41, thereby reducing the vibration and swing of the soft shaft 41 during operation. In some embodiments, the flat wire spring tube 42 and the flexible shaft 41 are both made of metal materials, such as forged stainless steel, a nickel-titanium alloy, forged cobalt-chromium-molybdenum alloy, etc. In addition, since the soft shaft 41 rotates at a high speed during operation, a lubricating fluid needs to be applied thereto to reduce friction and lower temperature. The lubricating fluid may be a liquid harmless to the human body such as physiological saline, distilled water or glucose solution. The sealing hose 43 may be made of a flexible polymer material such as polyurethane.

As shown in Fig. 5 and Fig. 6, in this embodiment, a distal end of the soft shaft 41 is fixed to the impeller shaft 33 rotatably arranged in an inner ring of the bearing 9, and a proximal end of the soft shaft is fixed to an output shaft 62 of the driving unit 6. The fixing may be achieved in any suitable manner, such as bonding, laser welding, crimping, clamping, etc. The pump body 101 further includes a tubular connector 10 fixed to the proximal ends of the outlet brackets 35. The tubular connector has a distal end region and a proximal end region, an outer diameter of the distal end region is greater than an outer diameter of the proximal end region, the distal end region is connected to a distal end of the sealing hose 43, and the proximal end region is connected to a distal end of the flat wire spring tube 42. In some embodiments, the tubular connector 10 is also made of a rigid material. In this way, the entire distal end of the flexible shaft 4 is connected to the rigid body, which can be well supported and improve the stability.

In the embodiment described above, the driving unit 6 is a motor, as shown in Fig. 6, in some embodiments, the motor includes a distal end cover extension section 61 and a motor connector 63 connecting the distal end cover extension section 61 and a proximal end of the flat wire spring tube 42. A proximal end of the sealing hose 43 is connected to the distal end cover extension section 61 of the motor. The distal end cover extension section 61, the motor connector 63, and the flat wire spring tube 42 of the motor may all be made of metal, and the above connection is achieved by laser welding, bonding, etc. According to one embodiment, the motor connector 63 and the distal end cover extension section 61 of the motor are integrally formed. According to another embodiment, before the flexible shaft 4 is connected to the motor, the length of the motor connector 63 extending from the distal end cover extension section 61 to the distal end is able to be adjusted. After adjusting the extension length according to the length relationship between the soft shaft 41, the flat wire spring tube 42 and the sealing hose 43, the motor connector 63 may be fixed to the distal end cover extension section 61 of the motor and the flat wire spring tube 42 by laser welding or bonding, etc.

In the embodiment shown in Fig. 1 to Fig. 6, the pump body 101 further includes a radially expandable catheter 5 arranged outside the flexible shaft 4 and extending in at least one part of the length of the flexible shaft 4, a distal end of the radially expandable catheter 5 is hermetically connected to the impeller outer cylinder 34 and covers the impeller outlet 36, and a proximal end of the radially expandable catheter is provided with the blood outlets 51. Specifically, the distal end of the radially expandable catheter 5 may be fixed to the outer surface of the proximal end of the impeller outer cylinder 34 by gluing, heat welding, etc. In order to increase the connection strength, a groove may be formed in the outer surface of the impeller outer cylinder 34, and surface treatments such as sandblasting, knurling, and threading may be performed in the groove. The blood outlets 51 are a plurality of openings uniformly distributed in the circumferential direction and formed in a proximal end wall of the radially expandable catheter 5, the shape of the blood outlets may be, for example, circular, elliptical, etc., and the number is usually 3 to 6. When the radially expandable catheter 5 is in a radially expandable state, the gap 52 between the radially expandable catheter and the flexible shaft 4 forms a blood flow channel communicating with the blood inlet 23 and the blood outlets 51. When the pump body 101 is at the operating position in the heart, the radially expandable catheter 5 spans the corresponding artery flap, so that the blood inlet 23 is located in the left ventricle or right ventricle, and the blood outlets 51 are located in the aorta or pulmonary artery. In some embodiments, as shown in Fig. 1, Fig. 2, and Fig. 6, the driving unit 6 is just arranged outside the blood outlets 51 and adjacent to the blood outlets 51, and the proximal end of the radially expandable catheter 5 is hermetically connected to a housing of the driving unit 6. In order to improve the connection strength, surface treatment may be performed on an outer surface of the housing of the driving unit 6 by sandblasting, grinding or rolling, etc. The radially expandable catheter 5 is made of flexible materials, and the flexible materials include, but are not limited to, flexible polymer materials, for example, made of one or more materials of Fluorinated Ethylene Propylene (FEP), Polyethylene Terephthalate (PET), Expanded Polytetrafluoroethylene (E-PTFE), polyurethane, nylon, polyether block polyamide, and latex. During surgical intervention, the radially expandable catheter 5 is in a contracted state and tightly attached to an outer wall of the flexible shaft 4 to reduce the diameter, so that the size is relatively small during intervention, which can reduce vascular damage, and facilitate the reduction of vascular complications and postoperative recovery. During operation, as the impeller 3 rotates, the blood is pumped into the gap between the impeller 3 and the impeller outer cylinder 34 from the blood inlet 23 in the inlet bracket 2, flows through the gap and flows out from the impeller outlet 36, and enters the gap 52 between a wall of the radially expandable catheter 5 and the flexible shaft 4. As the blood continues to enter, the wall of the radially expandable catheter 5 gradually expands radially until the radially expandable catheter is in an operating state with a relatively large diameter. The blood flows through the blood flow channel formed by the gap 52 and flows out from the blood outlets 51 at the proximal end of the radially expandable catheter 5 and enters the artery. Since the inner diameter of the radially expandable catheter 5 may be expanded during operation of the blood pump, and the blood flow channel with a relatively large cross-section is formed between the expandable catheter and the flexible shaft 4, the flow area of the blood can be ensured. In addition, since the blood flowing out from the impeller outlet 36 does not flow directly into the artery, but enters the artery from the blood outlet 51 after passing through the blood flow channel. Research has found that such an arrangement prevents the impeller outlet from dissipating outlet energy due to blood flow chaos, thereby reducing pressure loss and improving the operating efficiency of the entire pump.

A pump head of the interventional blood pump 100 described above with reference to Fig. 1 to Fig. 6 may be delivered to the body of the patient through a guide wire or a sheath tube, and the pressure difference detection and/or medical imaging are/is used to comprehensively determine whether the pump head is placed at the desired position. When the sheath tube is used for delivery, during surgical intervention, the pump body 1 is in a radially constrained state under a radial constraint force applied by the sheath tube. At this time, the inlet bracket 2 and the radially expandable catheter 5 are both in a folded state to ensure that the blood vessel is intervened with a relatively small diameter. When it is determined that the blood inlet 23 has been delivered to the ventricle and the blood outlets 51 remain in the artery, that is, the radially expandable catheter 5 and the flexible shaft 4 accommodated therein span the artery flap, and the sheath tube is removed. At this time, the inlet bracket 2 may automatically restore the pre-set shape using its own memory characteristics and expand meshes thereof to a normal operating state. Subsequently, the motor is started to drive the impeller 3 to rotate, and the blood is pumped into the suction flow channel between the impeller 3 and the impeller outer cylinder 34 through the blood inlet 23 (i.e., the meshes of the inlet bracket 2). Then, the blood enters the gap between the wall of the radially expandable catheter 5 and the flexible shaft 4 from the impeller outlet 36, so that the radially expandable catheter 5 expands outward to form the blood flow channel with a relatively large cross-section. After passing through the blood flow channel, the blood enters the artery from the blood outlets 51 at the proximal end of the radially expandable catheter 5. When the blood pump completes the operation and needs to be removed from the body of the patient, the pump body 101 can be folded using the sheath tube, and the pump body 101 is withdrawn outside the body in the folded state.

According to another embodiment of the present invention, the length of the flexible shaft 4 is set, so that when the pump body 101 is at the operating position in the heart, the driving unit 6 is located outside the body of the patient. In this case, one part of the flexible shaft 4 is located inside the blood vessel, and one part is located outside the blood vessel. The radially expandable catheter 5 only covers one part of the length of the flexible shaft 4, so that the proximal end of the radially expandable catheter is not fixed to the housing of the driving unit, but to the outer surface of the flexible shaft 4. When the driving unit is arranged outside, there is no need to consider the problems of size, heat dissipation, material, etc. of the driving unit, which has greater design freedom and a relatively simple technical route. The size of the pump body part in the body may not be limited by the size of the motor.

The drawings and the above description describe non-limiting specific embodiments of the present invention. In order to teach the principles of the present invention, some conventional aspects have been simplified or omitted. The present invention is not limited to the above specific embodiments, but is only limited by the claims.

## Claims

1. An interventional blood pump, comprising a pump body (101) and a driving unit (6), the pump body (101) comprising a blood inlet (23) and a blood outlet (51), wherein the pump body comprises:
a rotatable rigid impeller (3) configured to increase power for the flow of blood and accommodated in a rigid impeller outer cylinder (34), a proximal end of the impeller (3) being connected to a distal end of the driving unit (6) through a flexible shaft (4), wherein a distal end of the impeller outer cylinder (34) communicates with the blood inlet (23), and a proximal end of the impeller outer cylinder (34) comprises an impeller outlet (36); and
a radially expandable catheter (5) arranged outside the flexible shaft (4) and extending in at least one part of a length of the flexible shaft (4), a distal end of the radially expandable catheter (5) being hermetically connected to the impeller outer cylinder (34) and covering the impeller outlet (36), and a proximal end of the radially expandable catheter being provided with the blood outlet (51); when the radially expandable catheter (5) is in a radial expansion state, a gap (52) between the radially expandable catheter and the flexible shaft (4) forms a blood flow channel communicating with the blood inlet (23) and the blood outlet (51);
wherein when the pump body (101) is at an operating position in the heart, the blood inlet (23) is located in the left ventricle or right ventricle, the blood outlet (51) is located in the aorta or pulmonary artery, and the radially expandable catheter (5) spans a corresponding artery flap;
**characterized in that**
the flexible shaft (4) comprises a soft shaft (41), a flat wire spring tube (42) sleeved outside the soft shaft (41), and a sealing hose (43) sleeved outside the flat wire spring tube (42),
wherein an inner diameter of the flat wire spring tube (42) is greater than an outer diameter of the soft shaft (41), and the outer diameter of the flat wire spring tube (42) is smaller than an inner diameter of the sealing hose;
the pump body (101) further comprises a plurality of rigid outlet brackets (35) connected to the proximal end of the impeller outer cylinder (34) and a tubular connector (10) fixed to proximal
ends of the outlet brackets (35), wherein the tubular connector has a distal end region and a proximal end region, an outer diameter of the distal end region is greater than an outer diameter of the proximal end region, the distal end region is connected to a distal end of the sealing hose, and the proximal end region is connected to a distal end of the flat wire spring tube (42);
wherein the driving unit (6) is a motor, and the motor comprises a distal end cover extension section (61) and a motor connector (63) connecting the distal end cover extension section (61) and a proximal end of the flat wire spring tube (42), wherein a length of the motor connector (63) extending from the distal end cover extension section (61) to the distal end is adjusted, and a proximal end of the sealing hose (43) is connected to the distal end cover extension section (61) of the motor.

2. The interventional blood pump according to claim 1, wherein a length of the flexible shaft (4) is set, so that when the pump body (101) is at the operating position in the heart, the driving unit is located in the aorta or pulmonary artery.

3. The interventional blood pump according to claim 1 or 2, wherein the driving unit (6) is arranged outside the expandable catheter (5) and adjacent to the blood outlet (51).

4. The interventional blood pump according to any one of claims 1 to 3, wherein the proximal end of the radially expandable catheter (5) is hermetically connected to a housing of the driving unit (6).

5. The interventional blood pump according to any one of claims 1 to 4, wherein the pump body (101) further comprises a plurality of rigid outlet brackets (35) connected to the proximal end of the impeller outer cylinder (34), wherein the outlet brackets (35) define the impeller outlet (36), and when the impeller (3) rotates, the blood entering from the blood inlet flows through a gap between the impeller (3) and the impeller outer cylinder (34), flows into the blood flow channel through the impeller outlet (36), and flows out from the blood outlet (51).

6. The interventional blood pump according to claim 5, wherein the pump body (101) further comprises a pigtail catheter (1) and a mesh inlet bracket (2), wherein the mesh inlet bracket (2) made of a shape memory alloy is arranged between a proximal end of the pigtail catheter (1) and the distal end of the impeller outer cylinder (34).

7. The interventional blood pump according to claim 6, wherein an outer diameter of the mesh inlet bracket (2) gradually decreases from a distal end to a proximal end and is slightly greater than outer diameters of the impeller outer cylinder (34) and the outlet brackets (35).

8. The interventional blood pump according to claim 6 or 7, wherein the mesh inlet bracket (2) further comprises a rigid ring (22) integrally formed therewith and fixed to the impeller outer cylinder (34) at the proximal end of the mesh inlet bracket.

9. The interventional blood pump according to any one of claims 5 to 8, wherein the pump body (101) further comprises a bearing seat (37) fixed to a proximal end of the outlet bracket (35), a bearing (9) being arranged in the bearing seat (37); and the impeller (3) comprises an impeller body (31), blades (32) arranged on an outer surface of the impeller body (31), and a rigid impeller shaft (33) extending from the impeller body (31) to the proximal end, the blades being rigid, the impeller shaft (33) being rotatably arranged in an inner ring of the bearing (9) in a penetrating manner.

10. **The** interventional blood pump according to claim 9, wherein the impeller body (31), the blades (32), and the impeller shaft (33) are fixed together to form a whole structure.

11. **The** interventional blood pump according to claim 9, wherein the blades (32) are made of implant grade metal materials or implant grade plastics.

12. **The** interventional blood pump according to any one of claims 1 to 11, wherein a length of the flexible shaft (4) is 50 mm to 80 mm.

13. **The** interventional blood pump according to any one of claims 1 to 12, wherein a distal end of the soft shaft (41) is fixed to the impeller shaft, and a proximal end of the soft shaft is fixed to an output shaft of the driving unit (6).

14. **The** interventional blood pump according to any one of claims 1 to 13, further comprising an interventional catheter (7) that is hollow, wherein a distal end of the interventional catheter (7) is connected to a proximal end of the driving unit (6), and the interventional catheter (7) contains at least a cable for supplying power to the driving unit (6).

## Patentansprüche

1. Interventionelle Blutpumpe, umfassend einen Pumpenkörper (101) und eine Antriebseinheit (6), wobei der Pumpenkörper (101) einen Bluteinlass (23) und einen Blutauslass (51) umfasst, wobei der Pumpenkörper Folgendes umfasst:
ein drehbares, starres Laufrad (3), das dazu konfiguriert ist, die Leistung für den Blutfluss zu steigern, und in einem Außenzylinder (34) des starren Laufrads untergebracht ist, wobei ein proximales Ende des Laufrads (3) über eine flexible Welle (4) mit einem distalen Ende der Antriebseinheit (6) verbunden ist, wobei ein distales Ende des Laufrad-Außenzylinders (34) mit dem Bluteinlass (23) in Verbindung steht und ein proximales Ende des Laufrad-Außenzylinders (34) einen Laufradauslass (36) umfasst; und
einen radial aufweitbaren Katheter (5), der außerhalb der flexiblen Welle (4) angeordnet ist und sich über mindestens einen Teil einer Länge der flexiblen Welle (4) erstreckt, wobei ein distales Ende des radial aufweitbaren Katheters (5) hermetisch mit dem Laufrad-Außenzylinder (34) verbunden ist und den Laufradauslass (36) abdeckt und ein proximales Ende des radial aufweitbaren Katheters mit dem Blutauslass (51) versehen ist; wenn sich der radial aufweitbare Katheter (5) in einem radial aufgeweiteten Zustand befindet, ein Spalt (52) zwischen dem radial aufweitbaren Katheter und der flexiblen Welle (4) einen Blutflusskanal bildet, der mit dem Bluteinlass (23) und dem Blutauslass (51) in Verbindung steht;
wobei, wenn sich der Pumpenkörper (101) an einer Betriebsposition in dem Herzen befindet, sich der Bluteinlass (23) in dem linken Ventrikel oder rechten Ventrikel befindet, sich der Blutauslass (51) in der Aorta oder Pulmonalarterie befindet und der radial aufweitbare Katheter (5) eine entsprechende Arterienklappe überspannt;
**dadurch gekennzeichnet, dass**
die flexible Welle (4) eine weiche Welle (41), ein Flachdraht-Federrohr (42), das um die weiche Welle (41) herumgeschoben ist, und einen Dichtungsschlauch (43), der um das Flachdraht-Federrohr (42) herumgeschoben ist, umfasst, wobei ein Innendurchmesser des Flachdraht-Federrohrs (42) größer ist als ein Außendurchmesser der weichen Welle (41) und der Außendurchmesser des Flachdraht-Federrohrs (42) kleiner ist als ein Innendurchmesser des Dichtungsschlauchs;
der Pumpenkörper (101) ferner eine Vielzahl von starren Auslasshalterungen (35), die mit dem proximalen Ende des Laufrad-Außenzylinders (34) verbunden sind, und einen rohrförmigen Anschluss (10), der an proximalen Enden der Auslasshalterungen (35) befestigt ist, umfasst, wobei der rohrförmige Anschluss einen distalen Endbereich und einen proximalen Endbereich aufweist, ein Außendurchmesser des distalen Endbereichs größer ist als ein Außendurchmesser des proximalen Endbereichs, der distale Endbereich mit einem distalen Ende des Dichtungsschlauchs verbunden ist und der proximale Endbereich mit einem distalen Ende des Flachdraht-Federrohrs (42) verbunden ist;
wobei die Antriebseinheit (6) ein Motor ist und der Motor einen Verlängerungsabschnitt (61) der distalen Endabdeckung sowie einen Motoranschluss (63), der den Verlängerungsabschnitt (61) der distalen Endabdeckung mit einem proximalen Ende des Flachdraht-Federrohrs (42) verbindet, umfasst, wobei eine Länge des Motoranschlusses (63), die sich von dem Verlängerungsabschnitt (61) der distalen Endabdeckung bis zu dem distalen Ende erstreckt, eingestellt ist und ein proximales Ende des Dichtungsschlauchs (43) mit dem Verlängerungsabschnitt (61) der distalen Endabdeckung des Motors verbunden ist.

2. Interventionelle Blutpumpe nach Anspruch 1, wobei eine Länge der flexiblen Welle (4) derart festgelegt ist, dass, wenn sich der Pumpenkörper (101) an der Betriebsposition in dem Herzen befindet, sich die Antriebseinheit in der Aorta oder Pulmonalarterie befindet.

3. Interventionelle Blutpumpe nach Anspruch 1 oder 2, wobei die Antriebseinheit (6) außerhalb des aufweitbaren Katheters (5) und angrenzend an den Blutauslass (51) angeordnet ist.

4. Interventionelle Blutpumpe nach einem der Ansprüche 1 bis 3, wobei das proximale Ende des radial aufweitbaren Katethers (5) hermetisch mit einem Gehäuse der Antriebseinheit (6) verbunden ist.

5. Interventionelle Blutpumpe nach einem der Ansprüche 1 bis 4, wobei der Pumpenkörper (101) ferner eine Vielzahl von starren Auslasshalterungen (35) umfasst, die mit dem proximalen Ende des Laufrad-Außenzylinders (34) verbunden sind, wobei die Auslasshalterungen (35) den Laufradauslass (36) definieren und, wenn sich das Laufrad (3) dreht, das von dem Bluteinlass eintretende Blut durch einen Spalt zwischen dem Laufrad (3) und dem Laufrad-Außenzylinder (34) fließt, durch den Laufradauslass (36) in den Blutflusskanal fließt und an dem Blutauslass (51) herausfließt.

6. Interventionelle Blutpumpe nach Anspruch 5, wobei der Pumpenkörper (101) ferner einen Pigtail-Katheter (1) und eine Gitter-Einlasshalterung (2) umfasst, wobei die Gitter-Einlasshalterung (2), die aus einer Formgedächtnislegierung gefertigt ist, zwischen einem proximalen Ende des Pigtail-Katheters (1) und dem distalen Ende des Laufrad-Außenzylinders (34) angeordnet ist.

7. Interventionelle Blutpumpe nach Anspruch 6, wobei ein Außendurchmesser der Gitter-Einlasshalterung (2) von einem distalen Ende zu einem proximalen Ende hin allmählich abnimmt und geringfügig größer ist als Außendurchmesser des Laufrad-Außenzylinders (34) und der Auslasshalterungen (35).

8. Interventionelle Blutpumpe nach Anspruch 6 oder 7, wobei die Gitter-Einlasshalterung (2) ferner einen starren Ring (22) umfasst, der integral mit ihr ausgebildet und an dem Laufrad-Außenzylinder (34) an dem proximalen Ende der Gitter-Einlasshalterung befestigt ist.

9. Interventionelle Blutpumpe nach einem der Ansprüche 5 bis 8, wobei der Pumpenkörper (101) ferner einen Lagersitz (37), der an einem proximalen Ende der Auslasshalterung (35) befestigt ist, umfasst, wobei in dem Lagersitz (37) ein Lager (9) angeordnet ist; und das Laufrad (3) einen Laufradkörper (31), an einer Außenfläche des Laufradkörpers (31) angeordnete Schaufeln (32) und eine starre Laufradwelle (33), die sich von dem Laufradkörper (31) zu dem proximalen Ende erstreckt, umfasst, wobei die Schaufeln starr sind und die Laufradwelle (33) durchdringend drehbar in einem Innenring des Lagers (9) angeordnet ist.

10. Interventionelle Blutpumpe nach Anspruch 9, wobei der Laufradkörper (31), die Schaufeln (32) und die Laufradwelle (33) aneinander befestigt sind, um eine Gesamtstruktur zu bilden.

11. Interventionelle Blutpumpe nach Anspruch 9, wobei die Schaufeln (32) aus Metallwerkstoffen oder Kunststoffen in Implantatqualität hergestellt sind.

12. Interventionelle Blutpumpe nach einem der Ansprüche 1 bis 11, wobei eine Länge der flexiblen Welle (4) 50 mm bis 80 mm beträgt.

13. Interventionelle Blutpumpe nach einem der Ansprüche 1 bis 12, wobei ein distales Ende der weichen Welle (41) an der Laufradwelle befestigt ist und ein proximales Ende der weichen Welle an einer Auslasswelle der Antriebseinheit (6) befestigt ist.

14. Interventionelle Blutpumpe nach einem der Ansprüche 1 bis 13, ferner umfassend einen interventionellen Katheter (7), der hohl ist, wobei ein distales Ende des interventionellen Katheters (7) mit einem proximalen Ende der Antriebseinheit (6) verbunden ist und der interventionelle Katheter (7) mindestens ein Kabel zum Bereitstellen von Leistung für die Antriebseinheit (6) beinhaltet.

## Revendications

1. Pompe d'assistance circulatoire interventionnelle, comprenant un corps de pompe (101) et une unité d'entraînement (6), le corps de pompe (101) comprenant une entrée de sang (23) et une sortie de sang (51), dans laquelle le corps de pompe comprend :
un rotor rigide rotatif (3) configuré pour augmenter la puissance pour l'écoulement de sang et logé dans un cylindre externe de rotor rigide (34), une extrémité proximale du rotor (3) étant reliée à une extrémité distale de l'unité d'entraînement (6) par l'intermédiaire d'un arbre flexible (4), dans laquelle une extrémité distale du cylindre externe de rotor (34) communique avec l'entrée de sang (23), et une extrémité proximale du cylindre externe de rotor (34) comprend une sortie de rotor (36) ; et
un cathéter radialement expansible (5) agencé à l'extérieur de l'arbre flexible (4) et s'étendant dans au moins une partie d'une longueur de l'arbre flexible (4), une extrémité distale du cathéter radialement expansible (5) étant reliée hermétiquement au cylindre externe de rotor (34) et recouvrant la sortie de rotor (36), et une extrémité proximale du cathéter radialement expansible étant pourvue de la sortie de sang (51) ;
lorsque le cathéter radialement expansible (5) est dans un état d'expansion radiale, un espace (52) entre le cathéter radialement expansible et l'arbre flexible (4) forme un canal d'écoulement de sang communiquant avec l'entrée de sang (23) et la sortie de sang (51) ;
dans laquelle lorsque le corps de pompe (101) est à une position de fonctionnement dans le cœur, l'entrée de sang (23) est située dans le ventricule gauche ou le ventricule droit, la sortie de sang (51) est située dans l'aorte ou l'artère pulmonaire, et le cathéter radialement expansible (5) enjambe une valvule artérielle correspondante ;
**caractérisée en ce que**
l'arbre flexible (4) comprend un arbre souple (41), un tube à ressort en fil plat (42) emmanché à l'extérieur de l'arbre souple (41), et un tuyau d'étanchéité (43) emmanché à l'extérieur du tube à ressort en fil plat (42), dans laquelle un diamètre intérieur du tube à ressort en fil plat (42) est supérieur à un diamètre extérieur de l'arbre souple (41), et le diamètre extérieur du tube à ressort en fil plat (42) est inférieur à un diamètre intérieur du tuyau d'étanchéité ;
le corps de pompe (101) comprend en outre une pluralité de supports de sortie rigides (35) reliés à l'extrémité proximale du cylindre externe de rotor (34) et un connecteur tubulaire (10) fixé à des extrémités proximales des supports de sortie (35), dans laquelle le connecteur tubulaire a une région d'extrémité distale et une région d'extrémité proximale, un diamètre extérieur de la région d'extrémité distale est supérieur à un diamètre extérieur de la région d'extrémité proximale, la région d'extrémité distale est reliée à une extrémité distale du tuyau d'étanchéité, et la région d'extrémité proximale est reliée à une extrémité distale du tube à ressort en fil plat (42) ;
dans laquelle l'unité d'entraînement (6) est un moteur, et le moteur comprend une section d'extension de couvercle d'extrémité distale (61) et un connecteur de moteur (63) reliant la section d'extension de couvercle d'extrémité distale (61) et une extrémité proximale du tube à ressort en fil plat (42), dans laquelle une longueur du connecteur de moteur (63) s'étendant à partir de la section d'extension de couvercle d'extrémité distale (61) jusqu'à l'extrémité distale est ajustée, et une extrémité proximale du tuyau d'étanchéité (43) est reliée à la section d'extension de couvercle d'extrémité distale (61) du moteur.

2. Pompe d'assistance circulatoire interventionnelle selon la revendication 1, dans laquelle une longueur de l'arbre flexible (4) est définie, de sorte que lorsque le corps de pompe (101) est à la position de fonctionnement dans le cœur, l'unité d'entraînement est située dans l'aorte ou l'artère pulmonaire.

3. Pompe d'assistance circulatoire interventionnelle selon la revendication 1 ou 2, dans laquelle l'unité d'entraînement (6) est agencée à l'extérieur du cathéter expansible (5) et adjacente à la sortie de sang (51).

4. Pompe d'assistance circulatoire interventionnelle selon l'une quelconque des revendications 1 à 3, dans laquelle l'extrémité proximale du cathéter radialement expansible (5) est reliée hermétiquement à un boîtier de l'unité d'entraînement (6).

5. Pompe d'assistance circulatoire interventionnelle selon l'une quelconque des revendications 1 à 4, dans laquelle le corps de pompe (101) comprend en outre une pluralité de supports de sortie rigides (35) reliés à l'extrémité proximale du cylindre externe de rotor (34), dans laquelle les supports de sortie (35) définissent la sortie de rotor (36), et lorsque le rotor (3) tourne, le sang entrant à partir de l'entrée de sang s'écoule à travers un espace entre le rotor (3) et le cylindre externe de rotor (34), s'écoule dans le canal d'écoulement de sang à travers la sortie de rotor (36), et s'écoule à l'extérieur à partir de la sortie de sang (51).

6. Pompe d'assistance circulatoire interventionnelle selon la revendication 5, dans laquelle le corps de pompe (101) comprend en outre un cathéter en queue de cochon (1) et un support d'entrée en treillis (2), dans laquelle le support d'entrée en treillis (2) constitué d'un alliage à mémoire de forme est agencé entre une extrémité proximale du cathéter en queue de cochon (1) et l'extrémité distale du cylindre externe de rotor (34).

7. Pompe d'assistance circulatoire interventionnelle selon la revendication 6, dans laquelle un diamètre extérieur du support d'entrée en treillis (2) diminue progressivement à partir d'une extrémité distale jusqu'à une extrémité proximale et est légèrement supérieur à des diamètres extérieurs du cylindre externe de rotor (34) et des supports de sortie (35).

8. Pompe d'assistance circulatoire interventionnelle selon la revendication 6 ou 7, dans laquelle le support d'entrée en treillis (2) comprend en outre un anneau rigide (22) formé d'un seul tenant avec celui-ci et fixé au cylindre externe de rotor (34) au niveau de l'extrémité proximale du support d'entrée en treillis.

9. Pompe d'assistance circulatoire interventionnelle selon l'une quelconque des revendications 5 à 8, dans laquelle le corps de pompe (101) comprend en outre un siège de palier (37) fixé à une extrémité proximale du support de sortie (35), un palier (9) étant agencé dans le siège de palier (37) ; et le rotor (3) comprend un corps de rotor (31), des pales (32) agencées sur une surface extérieure du corps de rotor (31), et un arbre de rotor rigide (33) s'étendant à partir du corps de rotor (31) jusqu'à l'extrémité proximale, les pales étant rigides, l'arbre de rotor (33) étant agencé de manière rotative dans une bague intérieure du palier (9) d'une manière pénétrante.

10. Pompe d'assistance circulatoire interventionnelle selon la revendication 9, dans laquelle le corps de rotor (31), les pales (32) et l'arbre de rotor (33) sont fixés ensemble pour former une structure entière.

11. Pompe d'assistance circulatoire interventionnelle selon la revendication 9, dans laquelle les pales (32) sont constituées de matériaux métalliques de qualité implant ou de plastiques de qualité implant.

12. Pompe d'assistance circulatoire interventionnelle selon l'une quelconque des revendications 1 à 11, dans laquelle une longueur de l'arbre flexible (4) est de 50 mm à 80 mm.

13. Pompe d'assistance circulatoire interventionnelle selon l'une quelconque des revendications 1 à 12, dans laquelle une extrémité distale de l'arbre souple (41) est fixée à l'arbre de rotor, et une extrémité proximale de l'arbre souple est fixée à un arbre de sortie de l'unité d'entraînement (6).

14. Pompe d'assistance circulatoire interventionnelle selon l'une quelconque des revendications 1 à 13, comprenant en outre un cathéter interventionnel (7) qui est creux, dans laquelle une extrémité distale du cathéter interventionnel (7) est reliée à une extrémité proximale de l'unité d'entraînement (6), et le cathéter interventionnel (7) contient au moins un câble pour l'alimentation en énergie de l'unité d'entraînement (6).
